# FASCICULE DE BREVET EUROPEEN

(11) **EP 0 674 716 B1**
(45) Date de publication et mention de la délivrance du brevet: **02.09.1998**
(21) Numéro de dépôt: 94902825.2
(22) Date de dépôt: 16.12.1993
(51) Int. Cl.: C12N 15/86, C12N 9/12, A61K 48/00, C12N 5/10

(54) **VECTEURS RETROVIRAUX POUR LE TRAITEMENT DES TUMEURS, ET LIGNEES CELLULAIRES LES CONTENANT**
RETROVIRALE VEKTOREN ZUR TUMORBEHANDLUNG UND SIE ENTHALTENDE ZELLLINIEN
RETROVIRAL VECTORS FOR THE TREATMENT OF TUMORS, AND CELL LINES CONTAINING THEM

(30) Priorité: 16.12.1992 FR 9215195
(43) Date de publication de la demande: 04.10.1995
(73) Titulaire: UNIVERSITE PIERRE ET MARIE CURIE PARIS VI, 75252 Paris Cédex 05 (FR)
(72) Inventeur: KLATZMANN, David, F-75013 Paris (FR); CARUSO, Manuel, F-75018 Paris (FR)
(74) Mandataire: Desaix, Anne
(86) Numéro de dépôt international: FR9301259
(87) Numéro de publication internationale: WO9413824

(56) Documents cités:
- SCIENCE vol. 256, no. 5063 , 12 Juin 1992 , LANCASTER, PA US pages 1550 - 1551 CULVER, K. W. ET AL. 'In vivo gene transfer with retroviral vector-producer cells for treatment of experimental brain tumors' cité dans la demande
- PROCEEDINGS OF THE NATIONAL ACADEMY OF SCIENCES OF USA. vol. 90, no. 15 , Aoüt 1993 , WASHINGTON US pages 7024 - 7028 CARUSO, M. ET AL. 'Regression of established macroscopic liver metastases after in situ transduction of a suicide gene'
- Cell, vol. 47, 1988, pages 391-399

## Description

La thérapie génique offre de nouvelles possibilités pour le traitement des tumeurs.

Parmi les différentes options, le transfert de gène peut avoir pour but de tuer les cellules tumorales soit indirectement par l'induction ou le renforcement d'une réponse immunitaire de l'hôte (S.A. Rosenberg, Cancer Res. 51:5074 (1991)) ou directement par l'insertion d'un "gène suicide" (F.L. Moolten, Cancer Res. 46:52-76 (1986)). Le gène suicide le plus étudié est celui codant pour la thymidine kinase du virus Herpès Simplex de type 1 (TK-HSV1) (G.B. Elion et al., J. Antimicrob. Chemother. 12:9-17 (1983)). Cette enzyme permet la phosphorylation d'analogues nucléosidiques tels que le ganciclovir (GCV). Ces molécules monophosphates sont ensuite transformées en formes triphosphatées par les enzymes cellulaires. Le GCV triphopshate (GCV-TP) peut alors être incorporé dans l'ADN durant la division cellulaire, bloquant la l'élongation et entraînant ainsi la mort de la cellule. Le CGV-TP n'est donc toxique que pour les cellules en division.

Cette approche est particulièrement intéressante pour le traitement des tumeurs qui sont constituées de cellules en division rapide dans un tissu constitué de cellules non prolifératives, et l'expression de TK- HSV1 par les cellules d'une tumeur située au sein d'un organe dont les cellules ne se divisent pas devrait permettre la destruction spécifique de ces cellules. De plus, il est possible de cibler le transfert de gène sur ces cellules tumorales en utilisant pour la transduction de TK-HSV1 des rétrovirus recombinant dont le génome ne peut s'intégrer et s'exprimer que dans des cellules en division.
Le premier modèle expérimental d'ablation par le GCV de cellules tumorales exprimant TK-HSV1 a été réalisé par Moolten (F. L. MOOLTEN, Cancer Res, 46, 1986, p. 5276-5281) ; (F. L. MOOLTEN et J. M. WELLS, J. Natl. Cancer Inst., 82, 1990, p. 297-300). Il a montré un effet antitumoral du GCV sur la croissance de cellules tumorales de souris, après transfection du gène TK-HSV1 in vitro, et réimplantation chez l'animal. Récemment, l'élimination de tumeurs cérébrales expérimentales microscopiques par injection stéréotaxique de cellules produisant des rétrovirus TK-HSV1 et traitement par le GCV a été rapportée par Culver (K.W. Culver et coll., Science 256:1550-1552 (1992)).

Cependant, dans ce modèle les auteurs ne peuvent pas analyser l'effet d'un tel traitement sur des tumeurs établies macroscopiques qui sont rapidement létales. Or, chez les patients les tumeurs solides sont la cible principale de ce type de traitement et le problème de la transduction des gènes suicides dans le contexte d'une masse tumorale est beaucoup plus problématique.

Chez l'homme, les métastases hépatiques sont une complication fréquente des cancers digestifs. L'hépatectomie partielle n'est possible que dans environ 15% des cas, et les autres traitements comme la chimiothérapie loco-régionale ou l'immunothérapie n'ont donné jusqu'à présent que des résultats modestes, voire décevants. Par le présent travail, il est montré l'efficacité du traitement de métastases hépatiques expérimentales chez le rat après transfert in vivo du gène TK-HSV1 par injection directe de fibroblastes murins produisant des particules rétrovirales recombinantes.

Dans ce cadre, un des buts de la présente invention est la mise au point d'un système cellules de packaging-vecteur rétroviral recombinant utilisable en thérapeutique pour soigner les tumeurs établies, notamment les tumeurs hépatiques, et dans lequel le produit d'expression du gène recombiné est capable de transformer une prodrogue non toxique en drogue toxique pour les cellules exprimant ledit gène recombinant.

Plus précisemment la présente invention a pour but de construire un vecteur rétroviral recombinant susceptible, après transformation d'une lignée fibroblastique de packaging, de produire des pseudo-particules rétrovirales qui, après infection de cellules en division, aboutit à une expression du gène transduit, sans pour autant conduire à une inactivation desdits vecteurs comme cela se produit et a été décrit notamment avec le MuLV-moloney.

Il est usuel de dire que si les vecteurs rétroviraux sont des bons candidats comme vecteurs d'expression de gènes recombinés ou séquences hétérologues dans des cellules en division, cette expression présente une instabilité in vivo notamment quand les cellules sont peu différenciées,

Barklis et al, Cell (1986), 47 : 291-398, en cherchant à comprendre le mécanisme de rectriction de l'expression rétrovirale dans les cellules carcino-embryonnaires (C.C.E.), ont décrit un vecteur rétroviral capable de se répliquer dans des C.C.E. alors que la forme non mutante du même virus en est incapable.

Au moins, deux mécanismes différents ont ainsi été mis en évidence : le premier, largement prédominant, est lié au site d'intégration, et le deuxième, ne concernant qu'un seul provirus, est relatif à cette mutation qui, par ailleurs, est hautement réversible.

Néanmoins, cet article ne suggère pas que ce type de mutation pourrait fournir un moyen de maintenir l'expression rétrovirale dans des cellules en division. En effet, ces observations conduisent au contraire les auteurs à s'interroger sur la fonction du site de liaison au tRNA dans l'expression des rétrovirus.

L'étude de l'infectivité par des rétrovirus au stade embryonnaire de développement a mis en évidence (Jaenisch et al (1981) Cell 24 : 519-521) que certaines lignées transgéniques murines de la série Mov établies par infection d'embryons de souris par le virus de Moloney-MuLV avant implantation pourraient présenter des caractéristiques qui permettraient de surmonter les limites des vecteurs rétroviraux présentées ci-dessus.

Au stade embryonnaire de développement, les animaux sont normalement résistants à l'infection par le MuLV, l'hypothèse étant que l'ADN proviral subirait un processus de méthylation acquis au cours des divisions cellulaires successives et cette méthylation empêcherait la transcription de l'ADN. Cependant, avec certaines lignées Mov, le génome MuLV peut être réactivé à un stade particulier du développement et les rongeurs développent des leucémies induites par ce virus oncogène. Ceci est le cas pour les trois lignées suivantes : Mov3, Mov9 et Mov 13. Ce phénotype serait dû à des mutations dans le LTR.

La présente invention résulte de l'hypothèse selon laquelle certaines mutations dans des séquences rétrovirales contrôlant l'expression des gènes pourraient permettre une expression stable des rétrovirus dans des cellules en division, notamment des cellules cancéreuses ou des tumeurs établies.

La présente invention a donc pour but la construction d'un vecteur rétroviral recombinant dérivé du MuLV-moloney porteur d'un gène suicide susceptible de transformer une substance inactive en substance toxique pour des cellules en division, et caractérisé dans sa structure par la présence de séquences LTR issues de tels variants du MuLV ayant la propriété de ne pas être inactivés lors d'un passage dans des cellules carcinoembryonnaires ou lignées germinales de souris et d'être capables de transfecter des cellules qui se divisent et notamment des cellules tumorales.

Le gène suicide utilisé est celui de la thymidine kinase du virus Herpès Simplex.

L'invention concerne également des lignées de packaging transfectées par ce vecteur recombinant dérivé du rétrovirus de Moloney et comprenant le gène TK-HSV1.

L'invention concerne plus particulièrement la lignée M11 déposée à la C.N.C.M. le 15 décembre 1992 sous le n° I-1278.

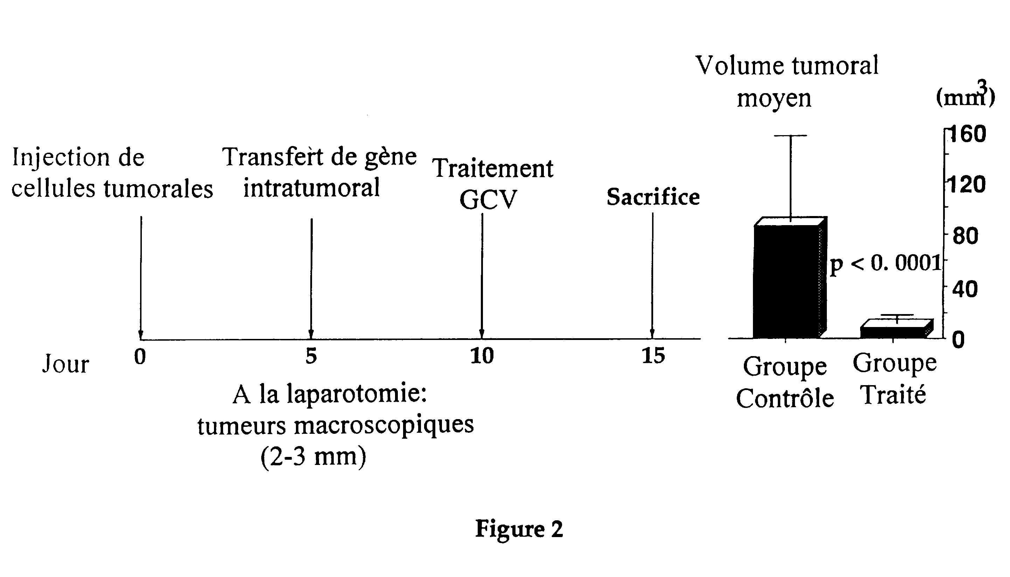

L'administration in situ dans une tumeur hépatique de ces lignées transfectées, porteuses de gène TK-HSV1 suivi par un traitement par un analogue de nucléotides tel le ganciclovir conduit à une réduction de la masse tumorale supérieure à 90% dans les conditions expérimentales décrites ci-dessous (cf. figure 2).

L'invention concerne enfin des compositions thérapeutiques contenant les lignées cellulaires transfectées par des rétrovirus recombinants, utilisable en association avec une prodrogue pour conduire a une destruction sélective des cellules tumorales.

L'invention met à profit trois phénomènes distincts :
1) l'injection de la composition dans des cellules en division, elles-mêmes environnées de cellules quiescentes ;
2) la présence dans cette composition de vecteurs rétroviraux recombinants qui n'infectent que les cellules en division, et par voie de conséquence, le gène ne s'exprime que dans ces cellules ;
3) l'expression du gène suicide ne tue que les cellules en division.

Autrement dit, toutes les cellules en division infectées par les rétrovirus recombinants, et exprimant le gène TK-HSV1 sont susceptibles d'être tuées après traitement par l'analogue nucléosidique, qui peut être ainsi transformé en nucléotide triphosphate et stopper l'élongation de l'ADN après son intégration dans la chaîne d'ADN en croissance.

Les cellules susceptibles dans ce phénomène sont donc
- les cellules tumorales cibles
- les fibroblastes transfectés, de la composition de l'invention.

Les conditions expérimentales ci-après ont pour but d'illustrer la construction du vecteur rétroviral permettant la sélectivité de l'effet "tueur" décrit ci-dessus, la préparation des lignées transfectées par un tel vecteur, et l'efficacité thérapeutique de la composition contenant ces lignées, lorsque le traitement est associé à l'administration d'une prodrogue tel que le Ganciclovir.

### 1) Construction du vecteur rétroviral pMTK

On décrit la construction d'un vecteur dans lequel le gène HSV1-TK a été placé sous le contrôle d'un 5'LTR des souches MuLV mutantes Mov3, Mov13 et Mov9.

L'utilisation de ce plasmide pour la transfection des cellules fibroblastiques CRIP est décrite ensuite.

Les ligatures des différents fragments de restriction des souches Mov3, Mov13 et Mov9 sont réalisées de la façon suivante :
- LTR-5': - Mov3 jusqu'au site BamHI (-350 du départ de transcription).
- Mov13 du site BamHI (-350) jusqu'au site KpnI (+30).
- Mov9 du site KpnI (+30) jusqu'au site PstI (+560) (contient la séquence de packaging Ψ).
- La séquence non codante en 3' du site ClaI (+7674) jusqu'à U3 (+7817) provient de Mov3.
- LTR-3': - Mov3 jusqu'au site BamHI positionné en 7910 (ou -350).
- Mov13 jusqu'à la fin de U5.

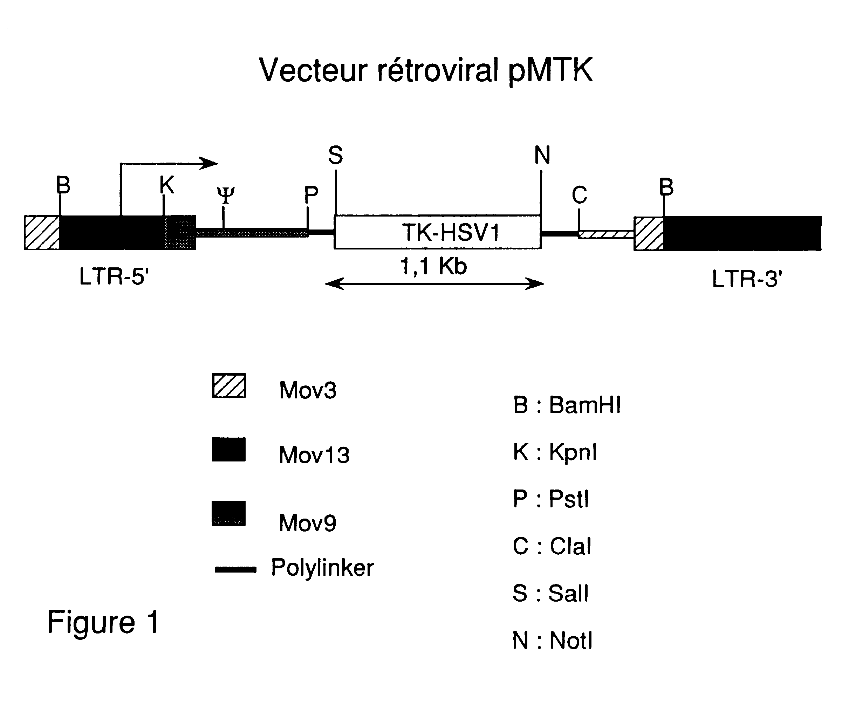

Le fragment contenant le gène HSV1-TK, de 1100 paires de bases, et délimité par les extrémités SalI et NotI a été inséré dans le polylinker aux sites indiqués dans la figure 1.

Toutes ces opérations de clonage ont été effectuées en utilisant des techniques classiques (Maniatis et al. (1982), Molecular Cloning, a laboratory manual).

La figure 1 représente le vecteur rétroviral recombinant.

### 2) Cultures cellulaires et transfection, établissement de la lignée packaging M11 :

Les cellules fibroblastiques Ψ CRIP (O. DANOS, et al, Proc. Natl. Acad. Sci. USA, (1988), 85 : 6460-64) ont été cotransfectées avec le vecteur rétroviral pMTK (20 µg) et exprimant TK-HSV1 sous le contrôle du LTR et un plasmide de sélection (1 µg)(pWLNeo, Stratagène). Plusieurs clones ont été isolés en sélection G418. La production de particules virales de ces clones a ensuite été testée par infection de cellules L TK- et sélection de cellules en HAT. L'infection se réalise avec des dilutions successives du surnageant contenant les particules virales, et le comptage du nombre de colonies obtenues dans ces conditions permet de déterminer un titre infectieux. Le clone M11 sélectionné possède un titre de 5.10⁵ particules virales/ml. La lignée NB16 (N. FERRY et al Proc. Natl. Acad. Sci. (1991) 88 : 8377-81), également dérivée de cellules Y CRIP, a été utilisée pour évaluer l'infection des cellules tumorales. Elle produit des particules virales exprimant le gène nls-LacZ avec un titre infectieux de 104 particules virales/ml.

La lignée DHDK12 est une lignée cellulaire établie de cancer colique chimio-induit chez le rat BDIX, décrite dans Martin et al., Virchows Archiv. A Pathol. Anat. (1991) 418:193.

L'invention concerne également les populations de cellules fibroblastiques transformées par un vecteur rétroviral recombinant porteur du gène suicide.

### 3) Protocole thérapeutique

Des rats BDIX mâles syngéniques ont été utilisés. Une tumeur de foie isolée a été induite par une injection directe des cellules DHDK12 sous la capsule du foie. Au jour 5 les tumeurs du foie ont un diamètre compris entre 2 et 3 mm et contiennent approximativement 150 millions de cellules.

A ce moment, les rats reçoivent une injection intra-tumorales des fibroblastes producteurs des rétrovirus recombinants (20.10⁶ cellules).

Les animaux du groupe contrôle ont reçu des cellules NB16 des fibroblastes qui produisent un rétrovirus recombinant exprimant le nls-lacZ gène. Ce gène code pour une β-galactosidase avec un signal de localisation dans le noyau et peut ainsi servir comme un gène indicateur de suivi de l'infection des cellules tumorales in vivo. Le clone NB16 produit le virus avec un titre de 10⁴ unités formant plaques par millilitre. Le groupe d'animaux traités ont été infectés par la lignée M11 qui produit 5.10⁵ particules infectieuses par millilitre.

Après l'injection intra-tumorale des cellules packaging, une période de 5 jours est laissée pour que l'infection des cellules tumorales par le rétrovirus recombinant puisse se produire.

Cinq jours plus tard, soit le dizième jour, tous les rats reçoivent du Ganciclovir (GCV) (Syntex), pendant cinq jours à la dose de 150 mg/kg deux fois par jour par voie intrapéritonéale et ce pendant 5 jours. Les rats étaient ensuite sacrifiés à la fin du traitement au Ganciclovir, soit 15 jours après la première injection et une autopsie a été réalisée comprenant une mesure de la taille des tumeurs et une étude anatomopathologique.

La figure 2 résume le protocole expérimental décrit ci-dessus et les résultats obtenus sur la réduction des tumeurs, décrits ci-dessous.

### RESULTATS

- Groupe contrôle (nombre de rats : 12) :
   Lors de l'autopsie, tous les lobes injectés présentaient des tumeurs macroscopiques, dont le diamètre moyen le plus large était de 6.5 ± 2.1 mm (extrêmes: 3 et 10 mm). Leur volume est calculé d'après la formule V = A x B²/2, dans laquelle A est le diamètre le plus large, et B le diamètre le plus petit mesuré au niveau de la section la plus large de la tumeur (G. Carlsson et al., 1983, J. Cancer Res. Clin. Oncol. 105:20). Le volume calculé dans ces conditions était de 86.3 ± 65.1mm³ (moyenne ± D.S.).
   L'examen pathologique des tumeurs révélait un aspect typique d'adénocarcinome faiblement différencié, consistant essentiellement en des cellules tumorales organisées en lobules d'épaisseurs variables. Le stroma apparaît fibrotique et entouré de cellules inflammatoires mononucléaires. Le pourcentage de cellules tumorales dans la tumeur peut être estimé à 60%.
   L'activité β-galactosidase nucléaire est détectée dans des coupes cryostatiques colorées Xdale chez les 6 foies témoins analysés. La fraction des cellules tumorales infectées était inférieure à 1%.
- Groupe traité (nombre de rats : 13) :
   Le diamètre moyen des tumeurs était de 3 plus ou moins 1.1 millimètre (extrêmes : 0.5 et 4 mm) et le volume était de 8.1 plus ou moins 6.7 mm³ (P < 0.0001, Mann and Withney Test) (Fig. 2). L'examen pathologique des tumeurs révèle l'efficacité de ce traitement par une diminution très importante de la taille des tumeurs. Sur les 11 foies analysées (2 étaient utilisées pour le contrôle analytique lacZ), deux avaient des tumeurs montrant une réduction de 60 à 20% des cellules tumorales. Ces cellules étaient faiblement organisées et nécrotiques. Elles étaient regroupées dans une réaction fibrotique entourant des cellules mononucléaires inflammatoires et une hyperplasie cancéreuse. Dans 5 autres foies, moins de 10% de cellules cancéreuses ont pu être détectées, avec une réaction fibrotique massive. Dans les quatre derniers foies, quelques rares cellules cancéreuses ont été vus et une nécrose massive de la tumeur entourée par une réaction fibrotique a été observée.

Chez tous ces rats, il y avait des signes d'une régénération parenchymateuse.

Ces études montrent une régression de tumeurs établies après transfert in vivo d'un gène suicide.

Chez des patients un ciblage précis de l'injection des fibroblastes packaging transformés dans la masse tumorale peut être facilitée par une assistance ultra sonographique ou par l'aide de toute microsonde dont la fonction est la localisation desdites masses tumorales.

Sachant que le taux de cellules infectées exprimant le gène TK-HSV1 était inférieur à 10% et que la tumeur est réduite de plus de 90% après addition de Ganciclovir, on peut supposer que le phénomène de "coopération métabolique", déjà décrit par Moolten (Moolten, 1986) ou "effet de proximité" (Culver, 1992) est également effectif dans le cas d'une masse tumorale importante. Ce phénomène pourrait s'expliquer par le transfert du ganciclovir phosphorylé des cellules exprimant le gène TK-HSV1 aux cellules ne l'exprimant pas.

L'utilisation des cellules fibroblastiques packaging, transformées par un vecteur rétroviral recombinant portant un gène suicide comme médicament associé à un traitement par le ganciclovir, paraît être efficace pour la réduction de masse tumorales et le traitement des cancers établis.

L'invention ne saurait être limitée au mode de réalisation qui a été illustré ci-dessus, en particulier la mise en oeuvre le couple TK-HSV et le Ganciclovir. L'homme du métier est à même d'imaginer d'autres couples actifs qui peuvent être mis en oeuvre à ces mêmes fins. On pourra citer par exemple comme gène tueur le gène de la thymidine kinase du cytomégalovirus couplé au Ganciclovir. La séquence génétique et la structure de la protéine de cette thymidine kinase montrent que celle-ci est relativement différente des kinases cellulaires ou de la kinase du virus Herpès Simplex 1. On peut citer également une enzyme bactérienne, la cytosine désaminase qui est capable de convertir la 5 fluorocytidine en un analogue de nucléotide toxique : le 5 fluoro-Uracyl. On peut enfin imaginer tout autre système de toxicité conditionnelle fondée sur un couple enzyme-médicament et qui permette par le système décrit ci-dessus de tuer spécifiquement les cellules en division.

## Revendications

1. Vecteur rétroviral recombinant contenant un gène thérapeutique dont l'expression dans des cellules tumorales est recherchée, ledit vecteur étant susceptible de produire des particules pseudo-rétrovirales après infection de cellules d'empaquetage, caractérisé en ce que les séquences rétrovirales contrôlant l'expression dudit gène sont choisies de telle façon que ladite expression ne soit pas inactivée lors d'un passage dans des cellules carcino-embryonnaires ou dans des lignées germinales de souris.

2. Vecteur selon la revendication 1, caractérisé en ce que le gène est un gène suicide, notamment le gène de la thymidine kinase de HSVI.

3. Vecteur selon la revendication 1 ou 2, caractérisé en ce que les séquences contrôlant l'expression du gène sont des séquences issues des variants Mov3 et/ou Mov9 et/ou Mov13.

4. Lignées cellulaires fibroblastiques d'empaquetage, caractérisées en ce qu'elles sont transformées par un vecteur rétroviral recombinant selon l'une des revendications 1 à 3 et productrices desdits virus recombinants.

5. Lignée cellulaire fibroblastique selon la revendication 4, caractérisée en ce qu'elle est transformée par un vecteur recombinant selon la revendication 3, et déposée à la C.N.C.M. le 15 décembre 1992 sous le n°I-1278.

6. Composition thérapeutique caractérisée en ce qu'elle contient une lignée cellulaire selon les revendications 4 ou 5, ou un vecteur rétroviral recombinant selon l'une des revendications 1 à 3.

7. Composition thérapeutique selon la revendication 6, caractérisée en ce que quand le gène est un gène suicide, la composition est associée à une pro-drogue.

8. Composition selon la revendication 7, caractérisée en ce que le vecteur porte le gène de la thymidine kinase du virus herpès simplex et la pro-drogue est un analogue de nucléotide tel le ganciclovir ou l'aciclovir.

9. Utilisation pour obtenir l'expression d'un gène thérapeutique dont l'expression est recherchée dans des cellules en division, d'un vecteur rétroviral dans lequel le gène dont l'expression est recherchée est sous le contrôle de séquences choisies de telle façon que ladite expression ne soit pas inactivée lors d'un passage dans des cellules carcino-embryonnaires ou dans des lignées germinales de souris.

10. Utilisation selon la revendication 9, caractérisée en ce que le gène est un gène suicide, notamment celui de la thymidine kinase de HSVI.

11. Utilisation selon la revendication 9 ou 10, caractérisée en ce que les séquences de contrôle du gène sont des séquences issues des variants Mov3 et/ou Mov9 et/ou Mov13.

12. Utilisation selon l'une quelconque des revendications 9 à 11, caractérisée en ce que les cellules en division sont des cellules tumorales.

## Patentansprüche

1. Rekombinanter retroviraler Vektor, welcher ein therapeutisches Gen umfaßt, dessen Expression in Tumorzellen erwünscht ist, wobei der Vektor nach Infektion von Hüllenzellen pseudo-retrovirale Partikel erzeugen kann, dadurch gekennzeichnet, daß die retroviralen Sequenzen, welche die Expression des Gens kontrollieren, so ausgewählt werden, daß die Expression während einer Passage in karzinoembryonalen Zellen oder in Maus-Keimlinien nicht inaktiviert wird.

2. Vektor nach Anspruch 1, dadurch gekennzeichnet, daß das Gen ein Suizid-Gen, insbesondere das Gen der Thymidin-Kinase von HSV1 ist.

3. Vektor nach Anspruch 1 oder 2, dadurch gekennzeichnet, daß die Sequenzen, welche die Expression des Gens kontrollieren, von den Varianten Mov3 und/oder Mov9 und/oder Mov13 abstammende Sequenzen sind.

4. Fibroblastische Hüllen-Zellinien, dadurch gekennzeichnet, daß sie durch einen rekombinanten retroviralen Vektor nach einem der Ansprüche 1 bis 3 transformiert werden und die rekombinanten Viren erzeugen.

5. Fibroblastische Zellinien nach Anspruch 4, dadurch gekennzeichnet, daß sie durch einen rekombinanten Vektor nach Anspruch 3 transformiert werden und am 15. Dezember 1992 unter der Nummer I-1278 bei C.N.C.M hinterlegt wurden.

6. Therapeutische Zusammensetzung, dadurch gekennzeichnet, daß sie eine Zellinie nach den Ansprüchen 4 oder 5 oder einen rekombinanten retroviralen Vektor nach einem der Ansprüche 1 bis 3 umfaßt.

7. Therapeutische Zusammensetzung nach Anspruch 6, dadurch gekennzeichnet, daß für den Fall, daß das Gen ein Suizid-Gen ist, die Zusammensetzung an eine Pro-Droge gebunden ist.

8. Zusammensetzung nach Anspruch 7, dadurch gekennzeichnet, daß der Vektor das Gen der Thymidin-Kinase des Herpes simplex-Virus trägt und die Pro-Droge ein Nukleotidanalog, wie Ganciclovir oder Aciclovir, ist.

9. Verwendung eines retroviralen Vektors, in welchem das Gen, dessen Expression erwünscht ist, sich unter Kontrolle von Sequenzen befindet, welche so ausgewählt werden, daß die Expression während einer Passage in karzinoembryonalen Zellen oder in Maus-Keimlinien nicht inaktiviert wird, um die Expression eines therapeutischen Gens zu erhalten, dessen Expression in sich teilenden Zellen erwünscht ist.

10. Verwendung nach Anspruch 9, dadurch gekennzeichnet, daß das Gen ein Suizid-Gen, insbesondere die Thymidin-Kinase von HSV1, ist.

11. Verwendung nach Anspruch 9 oder 10, dadurch gekennzeichnet, daß die Sequenzen zur Kontrolle des Gens von den Varianten Mov3 und/oder Mov9 und/oder Mov13 abstammende Sequenzen sind.

12. Verwendung nach irgendeinem der Ansprüche 9 bis 11, dadurch gekennzeichnet, daß die sich teilenden Zellen Tumorzellen sind.

## Claims

1. A recombinant retroviral vector containing a therapeutic gene the expression of which is desired in tumoral cells; said vector being capable of producing pseudo-retroviral particles after infection of packaging cells, characterized in that the retroviral sequences controlling expression of said gene are selected such that said expression is not inactivated during passage into carcino-embryonic cells or into mouse germ lines.

2. A vector according to claim 1, characterized in that the gene is a suicide gene, in particular the thymidine kinase gene of HSVI.

3. A vector according to claim 1 or claim 2, characterized in that the sequences controlling gene expression are sequences originating from variants Mov3 and/or Mov9 and/or Mov13.

4. Fibroblast packaging cell lines, characterized in that they are transformed by a recombinant retroviral vector according to any one of claims 1 to 3 and producers of said recombinant viruses.

5. A fibroblast cell line according to claim 4, characterized in that it is transformed by a recombinant vector according to claim 3, deposited at the C.N.C.M. on 15^{th} December 1992, registration n°I-1278.

6. A therapeutic composition, characterized in that it contains a cell line according to claim 4 or claim 5, or a recombinant retroviral vector according to any one of claims 1 to 3.

7. Therapeutic composition according to claim 6, characterized in that when the gene is a suicide gene, the composition is associated with a prodrug.

8. A composition according to claim 7, characterized in that the vector carries the thymidine kinase gene of the herpes simplex virus and the prodrug is a nucleotide analogue such as ganciclovir or aciclovir.

9. The use of a retroviral vector to obtain expression, in dividing cells, of a therapeutic gene the expression of which is desired, in which vector the gene for which expression is desired is under the control of sequences selected such that said expression is not inactivated during passage into carcino-embryonic cells or into mouse germ lines.

10. Use according to claim 9, characterized in that the gene is a suicide gene, in particular the thymidine kinase gene of HSV1.

11. Use according to claim 9 or claim 10, characterized in that the gene control sequences are sequences from variants Mov3 and/or Mov9 and/or Mov13.

12. Use according to any one of claims 9 to 11, characterized in that the dividing cells are turmoral cells.
